# EUROPEAN PATENT SPECIFICATION

(11) **EP 1 678 109 B1**
(45) Date of publication and mention of the grant of the patent: **19.01.2011**
(21) Application number: 04795224.7
(22) Date of filing: 14.10.2004
(51) Int. Cl.: C07C 29/16, C07C 31/02, C07C 31/125, B01J 12/00

(54) **PREPARATION OF BRANCHED ALIPHATIC ALCOHOLS USING A PROCESS STREAM FROM AN ISOMERIZATION UNIT WITH RECYCLE TO A DEHYDROGENATION UNIT**
HERSTELLUNG VON VERZWEIGTEN ALIPHATISCHEN ALKOHOLEN UNTER VERWENDUNG VON EINES VERFAHRENSSTROMS AUS EINER ISOMERISIERUNGSANLAGE MIT RÜCKFÜHRUNG ZU EINER DEHYDRIERUNGSANLAGE
PREPARATION D'ALCOOLS ALIPHATIQUES RAMIFIES A PARTIR D'UN CIRCUIT COMPRENANT UNE UNITE D'ISOMERISATION RELIEE A UNE UNITE DE DESHYDROGENATION

(30) Priority: 15.10.2003 US 511422 P
(43) Date of publication of application: 12.07.2006
(73) Proprietor: Shell Internationale Research Maatschappij B.V., 2596 HR Den Haag (NL)
(72) Inventor: AYOUB, Paul, Marie, NL-1031 CM Amsterdam (NL); DIRKZWAGER, Hendrik, NL-1031 CM Amsterdam (NL); MURRAY, Brendan, Dermot, Houston, TX 77077 (US); SUMROW, Steven, Clois, Katy, TX 77450 (US)
(74) Representative: Peereboom, Jan Hendrik Pieter Jacob
(86) International application number: PCT/US2004/034035
(87) International publication number: WO 2005/037751

(56) References cited:
- WO-A-00/12451
- WO-A-01/85654
- WO-A-02/064532
- US-A- 6 150 322

## Description

### Background of the Invention

### Field of Invention

The present invention generally relates to systems and methods for preparing aliphatic alcohols. More particularly, embodiments described herein relate to systems and methods for preparing branched aliphatic alcohols using an isomerization unit.

### Description of Related Art

Aliphatic alcohols are important compounds that may be used in a variety of applications or converted to other chemical compounds (e.g., surfactants, sulfates). Surfactants may be used in a variety of applications (e.g., detergents, soaps, oil recovery).

The structural composition of the aliphatic alcohol may influence the properties of the surfactant and/or detergent (e.g., water solubility, biodegradability and cold water detergency) produced from the aliphatic alcohol. For example, water solubility may be affected by the linearity of the aliphatic portion of the aliphatic alcohol. As the linearity of the aliphatic portion increases, the hydrophilicity (i.e., affinity for water) of the aliphatic alcohol surfactant may decrease. Thus, the water solubility and/or detergency performance of the aliphatic alcohol surfactant may decrease. Incorporating branches into the aliphatic portion of the aliphatic alcohol surfactant may increase the cold-water solubility and/or detergency of the aliphatic alcohol surfactant. Biodegradability, however, of the aliphatic alcohol surfactants may be reduced if the branches in the aliphatic portion of the alcohol surfactant include a high number of quaternary carbons. Incorporation of branches with a minimum number of quaternary carbon atoms into the aliphatic portion of the aliphatic alcohol surfactant may increase cold-water solubility and/or detergency of the alcohol surfactants while maintaining the biodegradability properties of the detergents.

The aliphatic portion of an aliphatic alcohol used to manufacture a surfactant may include one or more aliphatic alkyl groups as branches. Aliphatic alkyl groups that may form branches in the aliphatic portion may include methyl, ethyl, propyl or higher alkyl groups. Quaternary and tertiary carbons may be present when the aliphatic portion is branched. The number of quaternary and tertiary carbons may result from the branching pattern in the aliphatic portion. As used herein, the phrase "aliphatic quaternary carbon atom" refers to a carbon atom that is not bound to any hydrogen atoms.

Processes to manufacture branched primary alcohol compositions are described in U.S. Patent No. 5,849,960 to Singleton et al. entitled "Highly Branched Primary Alcohol Compositions, and Biodegradable Detergents Made Therefrom", U.S. Patent No. 6,150,322 to Singleton et al., entitled "Highly Branched Primary Alcohol Compositions, and Biodegradable Detergents Made Therefrom.", and in the International patent applications WO 02/064532, WO 01/85654 and WO 00/12451.

### Summary of the Invention

In an embodiment, aliphatic alcohols may be produced by a method that includes isomerization of the olefins. Isomerization of olefins in a process stream may occur in an isomerization unit. In certain embodiments, a process feed stream entering an isomerization unit is derived from a Fischer-Tropsch process. At least a portion of the linear olefins in a process feed stream may be isomerized to branched olefins in the isomerization unit. The resulting branched olefins may have an average number of branches per olefin molecule from 0.7 to 2.5. The branched olefins may include, but are not limited to, methyl and/or ethyl branched olefins. The isomerization process may produce branched olefins that include less than 0.5 percent of aliphatic quaternary carbon atoms.

After the feed stream is processed in the isomerization unit, the resulting branched olefin-containing stream is passed into a hydroformylation unit. One or more hydrocarbon streams may be combined with the branched olefin-containing stream to alter the concentration of olefins entering the hydroformylation unit. After hydroformylation of the olefins, unreacted components from the hydroformylation process may be separated from the aliphatic alcohol products. Paraffins and unreacted olefins in the separated stream may be sent to a dehydrogenation unit.

Dehydrogenation of paraffins may occur in a dehydrogenation unit. In an embodiment, at least a portion of a paraffins and unreacted olefins stream may enter a dehydrogenation unit. In the dehydrogenation unit, at least a portion of the paraffins in the paraffins and unreacted olefins stream may be dehydrogenated to produce olefins. At least a portion of the produced olefins may exit the dehydrogenation unit to form an olefinic hydrocarbon stream. The resulting olefinic hydrocarbon stream from the dehydrogenation process may be recycled back into the isomerization unit and/or into a stream entering the isomerization unit.

In an embodiment, one or more hydrocarbon streams may be combined with the feed stream entering an isomerization unit, a hydroformylation unit and/or a dehydrogenation unit. The hydrocarbon stream may be mixed with the feed stream to alter the concentration of the olefins entering the isomerization unit, hydroformylation unit and/or a dehydrogenation unit.

In certain embodiments, at least a portion of the aliphatic alcohols may be sulfated to form aliphatic sulfates. In some embodiments, aliphatic sulfates may include branched alkyl groups. In certain embodiments, at least a portion of the produced aliphatic alcohols may be oxyalkylated to form oxyalkyl alcohols. In some embodiments, oxyalkyl alcohols may include branched alkyl groups. In some embodiments, at least a portion of the produced branched aliphatic alcohols may be ethoxylated to form branched ethoxyalkyl alcohols. At least a portion of the oxyalkyl alcohols may be sulfated to from oxyalkyl sulfates. In some embodiments, oxyalkyl sulfates may include branched alkyl groups.

### Brief Description of the Drawings

Advantages of the present invention will become apparent to those skilled in the art with the benefit of the following detailed description of embodiments and upon reference to the accompanying drawings, in which:
FIG. 1 depicts a schematic diagram of an embodiment of a system for producing branched aliphatic alcohols using an olefin isomerization unit.
FIG. 2 depicts a schematic diagram of an embodiment of a system for producing branched aliphatic alcohols using a isomerization unit and a separation unit to separate branched olefins from linear olefins and paraffins.
FIG. 3 depicts a schematic diagram of an embodiment of a system for producing branched aliphatic alcohols using an olefin isomerization unit with addition of an additional hydrocarbon stream.

While the invention is susceptible to various modifications and alternative forms, specific embodiments thereof are shown by way of example in the drawing and will herein

### Detailed Description of Embodiments

Hydrocarbon products may be synthesized from synthesis gas (i.e., a mixture of hydrogen and carbon monoxide) using a Fischer-Tropsch process. Synthesis gas may be derived by partial combustion of petroleum (e.g., coal, hydrocarbons), by reforming of natural gas or by partial oxidation of natural gas. The Fischer-Tropsch process catalytically converts synthesis gas into a-mixture of products that includes saturated hydrocarbons, unsaturated hydrocarbons and a minor amount of oxygen-containing products. The products from a Fischer-Tropsch process may be used for the production of fuels (e.g., gasoline, diesel oil), lubricating oils and waxes.

Fischer-Tropsch process streams may also be used to prepare commodity products, which have economic value. For example, linear olefins are commodity products that are useful for the production of surfactants. Using a portion of the process stream to produce linear olefins may increase the economic value of a Fischer-Tropsch process stream.

Surfactants derived from branched olefins may have different properties than surfactants derived from linear olefins. For example, surfactants derived from branched olefins may have increased water solubility and/or improved detergency properties compared to surfactants derived from linear olefins. Biodegradable properties of the surfactant, however, may be affected by the presence of quaternary carbon atoms in the branched portion of the surfactant. Surfactants made from branched olefins with a minimum number of quaternary carbon atoms may have similar biodegradable properties to surfactants derived from linear olefins. Production of branched olefins from a Fischer-Tropsch process stream may increase the economic value of the stream. In some embodiments, linear olefins may be converted into branched olefins with a minimum ' number of quaternary carbon atoms using an isomerization catalyst. Increasing the amount of branched olefins derived from a Fischer-Tropsch process stream may increase the economic value of the process streams.

Methods are described for increasing the amount of branched olefins derived from a process stream that includes certain amount of olefins, thus increasing the economic value of the process stream. Such methods are useful for both Fischer-Tropsch process streams and product streams from other sources that include hydrocarbons.

A hydrocarbon feed stream composition may include paraffins and olefins. At least a portion of the hydrocarbon stream may be made up of linear paraffins and olefins having at least 4 carbon atoms and up to 18 carbon atoms. A hydrocarbon feed stream may be obtained from a Fischer-Tropsch process or from an ethylene oligomerization process. Fischer-Tropsch catalysts and reaction conditions may be selected to provide a particular mix of products in the reaction product stream. For example, a Fischer-Tropsch catalyst and reaction conditions may be selected to increase the amount of olefins and decrease the amount of paraffins and oxygenates in the stream. Alternatively, the catalyst and reaction conditions may be selected to increase the amount of paraffins and decrease the amount of olefins and oxygenates in the stream.

The catalyst used in a Fischer-Tropsch process may be Mo, W, Group VIII compounds or combinations thereof. Group VIII compounds include, but are not limited to, iron, cobalt, ruthenium, rhodium, platinum, palladium, iridium and osmium. Combinations of Mo, W and Group VIII compounds may be prepared in the free metal form. In an embodiment, combinations of Mo, W and Group VIII compounds may be formed as alloys. Combinations of Mo, W and Group VIII compounds may be formed, in some embodiments, as oxides, carbides or other compounds. In other embodiments, combinations of Mo, W and Group VIII compounds may be formed as salts. Iron based and cobalt based catalysts have been used commercially as Fischer-Tropsch catalysts. Ruthenium catalysts tend to favor the formation of high melting waxy species under highpressure conditions. Synthetic Fischer-Tropsch catalysts may include fused iron. In some embodiments, a fused iron Fischer-Tropsch catalyst may include a promoter (e.g., potassium or oxides on a silica support, alumina support or silica-alumina support). Cobalt metal may also be used in a Fischer-Tropsch catalyst. With the proper selection of supports, promoters and other metal combinations, a cobalt catalyst may be tuned to manufacture a composition enriched in the desired hydrocarbon species. Other catalysts, such as iron-cobalt alloy catalysts, are known for their selectivity toward the production of olefins. Catalysts and combinations for manufacture of hydrocarbon species by a Fischer-Tropsch process are generally known.

While reference is made to a Fischer-Tropsch stream, any stream of olefins and saturated hydrocarbons may be suitable. Many Fischer-Tropsch streams may contain from 5 percent to 80 percent olefins, the remainder being saturated hydrocarbons comprising paraffins and other compounds.

In some embodiments, feed streams containing olefins and paraffins are obtained through cracking of paraffin wax or the oligomerization of olefins. Commercial olefin products manufactured by ethylene oligomerization are marketed in the United States by Chevron Phillips Chemical Company, Shell Chemical Company (as NEODENE^{®}) and by British Petroleum. Cracking of paraffin wax to produce alpha-olefin and paraffin feed streams is described in U.S. Patent No. 4,579,986 to Sie, entitled "Process For The Preparation Of Hydrocarbons" and U.S. Patent Application Serial No. 10/153,955 of Ansorge et al., entitled "Process For The Preparation of linear Olefins and Use Thereof To Prepare Linear Alcohols." Specific procedures for preparing linear olefins from ethylene are described in U.S. Patent No. 3,676,523 to Mason, entitled "Alpha-Olefin Production;" U.S. Patent No. 3,686,351 to Mason, entitled "Alpha-Olefin Production;" U.S. Patent No. 3,737,475 to Mason, entitled "Alpha-Olefin Production" and U.S. Patent No. 4,020,121 to Kister et al., entitled "Oligomerization Reaction System." Most of the above-mentioned processes produce alpha-olefins. Higher linear internal olefins may be commercially produced (e.g., chlorination-dehydrochlorination of paraffins, paraffin dehydrogenation, isomerization of alpha-olefins).

In an embodiment, a feed stream is processed to produce a hydrocarbon stream that includes branched olefins. These branched olefins may be converted to branched aliphatic alcohols using various techniques. The feed stream may have a paraffin content range between 50 percent by weight to 90 percent by weight of the feed stream. In certain embodiments, a feed stream may have a paraffin content greater than 90 percent by weight paraffins. The feed stream may also include olefins. The olefin content of the feed stream may be between 10 percent by weight to 50 percent by weight. In other embodiments, a feed stream may have an olefin content greater than 90 percent by weight olefins.

The composition of the feed stream may include hydrocarbons having an average carbon number ranging from 4 to 30. In an embodiment, an average carbon number of the hydrocarbons in a feed stream may range from 4 to 24. In other embodiments, an average carbon number of the feed stream may range from 4 to 18. An average carbon number of the hydrocarbons in a feed stream may range from 7 to 18. In certain embodiments, an average carbon number of the hydrocarbons in a feed stream may range from 10 to 17. In some embodiments, an average carbon number of hydrocarbons in a feed stream may range from 10 to 13. In other embodiments, an average carbon number of hydrocarbons in a feed stream may range from 14 to 17.

In an embodiment, a feed stream for an isomerization unit includes mono-olefins and/or paraffins. The mono-olefins may be of a linear or branched structure. The mono-olefins may have an alpha or internal double bond position. The feed stream may include olefins in which 50 percent or more of the olefin molecules present may be alpha-olefins of a linear (straight chain) carbon skeletal structure. In certain embodiments, at least 70 percent of the olefins are alpha-olefins of a linear carbon skeletal structure. A hydrocarbon stream in which greater than 70 percent of all of the olefin molecules are alpha-olefins of a linear carbon skeletal structure may be used in certain embodiments to convert olefins to aliphatic alcohols. Such a stream may be derived from a Fischer-Tropsch process. In some embodiments, a feed stream includes olefins in which at least 50 percent of the olefin molecules present are internal olefins.

Branched chain olefins may be converted to branched aliphatic alcohols (e.g., branched primary alcohols) by a hydroformylation process. "Hydroformylation," as used herein, refers to the production of alcohols from olefins via a carbonylation and a hydrogenation process. Other processes may be used to produce aliphatic alcohols from olefins. Examples of other processes to produce aliphatic alcohols from olefins include, but are not limited to, hydration, oxidation and hydrolysis, sulfation and hydration, and epoxidation and hydration. The composition of an alcohol product stream may include aliphatic alcohols having an average carbon number ranging from 5 to 31. In an embodiment, an average carbon number of the aliphatic alcohols in an alcohol product stream may range from 7 to 20. In certain embodiments, an average carbon number of the aliphatic alcohols in an alcohol product stream may range from 11 to 18. In some embodiments, an average carbon number of aliphatic alcohols in an alcohol product stream may range from 11 to 14. In other embodiments, an average carbon number of aliphatic alcohols in an alcohol product stream may range from 15 to 18.

In certain embodiments, to reduce production costs of producing branched aliphatic alcohols, a stream containing a significant amount of paraffins and a minor amount of olefins may first be isomerized then hydroformylated to form branched aliphatic alcohols. Processing a stream containing a minor amount of olefins through an isomerization unit prior to hydroformylation may save production time, dehydrogenation catalyst cost and/or enhance the overall economic viability of the stream. In some embodiments, after hydroformylation, paraffins and unreacted olefins may be recycled to a dehydrogenation unit to produce a stream enriched in olefins. The enriched olefins stream may be recycled into an isomerization unit.

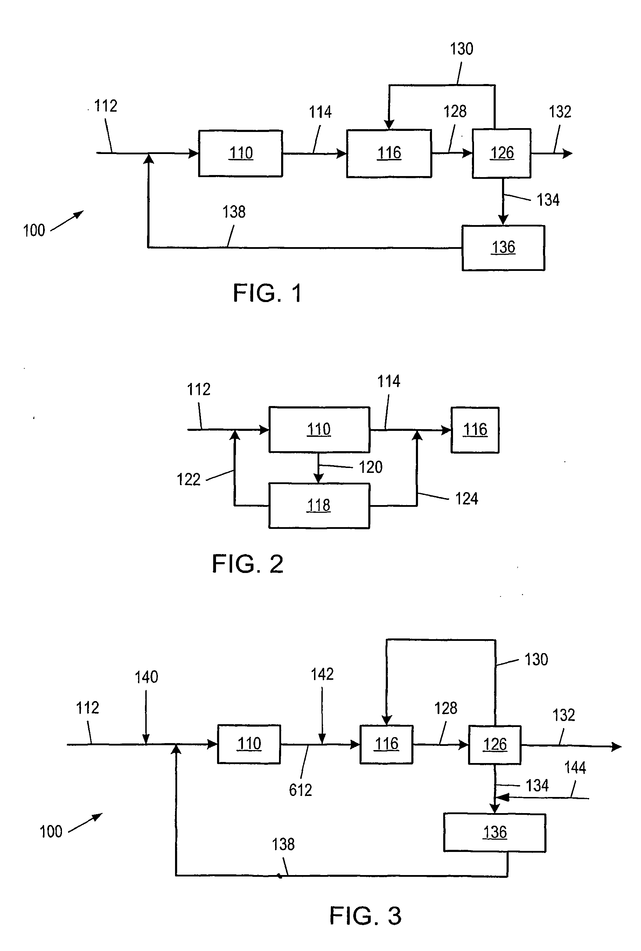

Referring to System 100 in FIG. 1, a first hydrocarbon stream may be introduced into isomerization unit 110 via first conduit 112. In isomerization unit 110, at least a portion of the olefins in the first hydrocarbon stream may be isomerized to branched olefins to produce a second hydrocarbon stream. Conditions for olefin isomerization in isomerization unit 110 may be controlled such that the number of carbon atoms in the olefins before and after the isomerization is substantially the same. Catalysts and process conditions to skeletally isomerize linear olefins to branched olefins are described in U. S. Patent No. 5,648,584 to Murray, entitled "Process for Isomerizing Linear Olefins to Isoolefins" and U.S. Patent No. 5,648,585 to Murray et al., entitled "Process for Isomerizing Linear Olefins to Isoolefins."

In an embodiment, linear olefins in a first hydrocarbon stream are isomerized in isomerization unit 110 by contacting at least a portion of the first hydrocarbon stream with a zeolite catalyst. The zeolite catalyst may have at least one channel with a crystallographic free channel diameter ranging from greater than 4.2 Å and less than 7 Å. The zeolite catalyst may have an elliptical pore size large enough to permit entry of a linear olefin and diffusion, at least partially, of a branched olefin. The pore size of the zeolite catalyst may also be small enough to retard coke formation.

Temperatures at which the olefin isomerization may be conducted range from 200 °C to 500 °C. Temperatures in isomerization unit 110 are, in some embodiments, kept below the temperature at which the olefin will crack extensively. To inhibit cracking, low temperatures may be used at low feed rates. In certain embodiments, lower temperatures may be used when the amount of oxygenates present in the process stream is low. Higher feed rates may be desirable to increase the production rate of isomerised products. Higher feed rates may be used, in some embodiments, when operating at higher reaction temperatures. The reaction temperature, however, should be set such that cracking to lower boiling weight products is minimized. For example, greater than 90 percent of linear olefins may be converted to branched olefins at 230 °C at a feed rate of 60 grams per hour with minimal cracking. Pressures maintained in isomerization unit 110 may be at a hydrocarbon partial pressure ranging from 0.1 atmosphere (10 kPa) to 20 atmospheres (2026 kPa). In an embodiment, partial pressure may range from above 0.5 atmosphere (51 kPa) to 10 atmospheres (1013 kPa).

The branched olefin produced in isomerization unit 110 may include methyl, ethyl and/or longer carbon chain branches. The isomerized olefin composition may be analyzed by ¹H NMR. In an embodiment, an average number of branches per olefin molecule present in the produced branched olefin composition may be greater than 0.7. In certain embodiments, an average number of branches per olefin molecule present in the branched olefin composition is from 0.7 to 2.5. In some embodiments, an average number of branches per olefin molecule present in the branched olefin composition is from 0.7 to 2.2. In certain embodiments, an average number of branches per olefin molecule present in the branched olefin composition is from 1.0 to 2.2. The degree of branching in the product may be controlled by controlling process conditions the isomerization unit. For example, high reaction temperatures and lower feed rates may result in a higher degree of branching. Methyl branches may represent between 20 percent to 99 percent of the total number of branches present in the olefin molecules. In some embodiments, methyl branches may represent greater than 50 percent of the total number of branches in the olefin molecules. The number of ethyl branches in the olefin molecules may represent, in certain embodiments, less than 30 percent of the total number of branches. In other embodiments, a number of ethyl branches, if present, may be between 0.1 percent and 2 percent of the total number of branches. Branches other than methyl or ethyl, if present, may be less than 5 percent of the total number of branches.

Isomerization unit 110 may produce a second hydrocarbon stream that includes olefins and paraffins. At least a portion of the second hydrocarbon stream contains branched olefins. The second hydrocarbon stream may exit isomerization unit 110 via second conduit 114 and be introduced into hydroformylation unit 116. At least a portion of the olefins in the second hydrocarbon stream may be hydroformylated.

In an embodiment, olefins may be separated, if desired, from the second hydrocarbon stream through techniques generally known in the art (e.g., distillation, molecular sieves, extraction, adsorption, adsorption/desorption, and/or membranes). Referring to FIG. 2, a second hydrocarbon stream may exit isomerization unit 110 and enter separation unit 118 via separation conduit 120. Separation unit 118 may produced at least two streams, a branched olefins stream and a linear olefins and paraffins stream. In separation unit 118, the second hydrocarbon stream may be contacted with molecular sieves (e.g., zeolite or urea) of the correct pore size for absorption of branched olefins and/or linear olefins and paraffins. Subsequent desorption of at least a portion of the branched olefins and/or at least a portion of the linear olefins and paraffins from the molecular sieves may produce at least two streams, a branched olefins stream and a linear olefins and paraffins stream.

Separation unit 118 may include a fixed bed containing adsorbent for separation of the second hydrocarbon stream to produce a branched olefin stream and a linear olefins and paraffins stream. Separation temperatures in separation unit 118 may range from 100°C to 400°C. In some embodiments, separation temperatures may range from 180 °C to 380°C. Separations in separation unit 118 may be conducted at a pressure ranging from 2 atmospheres (202 kPa) to 7 atmospheres (710 kPa). In some embodiments, a pretreatment of a second hydrocarbon stream may be performed to prevent adsorbent poisoning.

At least a portion of the linear olefins and paraffins stream may be recycled, transported to other processing units and/or stored on site. In an embodiment, at least a portion of the linear olefins and paraffins stream may be combined with first hydrocarbon stream in first conduit 112 via linear olefin and paraffin recycle conduit 122. The combined stream may enter isomerization unit 110 via first conduit 112 to continue the process to produce isomerized olefins. In some embodiments, a linear olefins and paraffins stream may be introduced directly into isomerization unit 110. In some embodiments, a linear olefins and paraffins stream may be introduced into a dehydrogenation unit.

At least a portion of the branched olefins stream may be transported and utilized in other processing streams and/or stored on site via branched olefins conduit 124. In some embodiments, at least a portion of a branched olefins stream may exit separation unit 118 and be combined with second hydrocarbon stream in second conduit 114 upstream of hydroformylation unit 116 via branched olefins conduit 124. In other embodiments, at least a portion of a branched olefins stream may exit a separation unit and be introduced directly into a hydroformylation unit.

The second hydrocarbon stream may exit isomerization unit 110 via second conduit 114 and enter hydroformylation unit 116 as depicted in FIGS. 1 and 2. In a hydroformylation process, olefins are converted to aldehydes, alcohols or a combination thereof by reaction of at least a portion of the olefins with carbon monoxide and hydrogen according to an Oxo process. As used herein, an "Oxo process" refers to the reaction of an olefin with carbon monoxide and hydrogen in the presence of a metal catalyst (e.g., a cobalt catalyst) to produce an alcohol containing one more carbon atom than the starting olefin. In other hydroformylation processes, a "modified Oxo process" is used. As used herein, a "modified Oxo process" refers to an Oxo process that uses a phosphine, phosphite, arsine or pyridine ligand modified cobalt or rhodium catalyst. Preparation and use of modified Oxo catalysts are described in U.S. Patent No. 3,231, 621, to Slaugh, entitled "Reaction Rates In Catalytic Hydrofomylation"; U.S. Patent No. 3,239,566 to Slaugh et al., entitled "Hydroformylation Of Olefins;" U.S. Patent No. 3,239,569 to Slaugh et al., entitled "Hydroformylation Of Olefins;" U.S. Patent No. 3,239,570 to Slaugh et al., entitled "Hydroformylation Of Olefins;" U.S. Patent No. 3,239,571 to Slaugh et al., entitled "Hydroformylation Of Olefins;" U.S. Patent No. 3,400,163 to Mason et al., entitled "Bicyclic Heterocyclic Sec- And Tert-Phosphines;" U.S. Patent No. 3,420,898 to Van Winkle et al., entitled "Single Stage Hydroformylation Of Olefins To Alcohols Single Stage Hydroformylation Of Olefins To Alcohols;" U.S. Patent No. 3,440,291 to Van Winkle et al., entitled "Single Stage Hydroformylation Of Olefins To Alcohols;" U.S. Patent No. 3,448,157 to Slaugh et al., entitled "Hydroformylation Of Olefins;" U.S. Patent No. 3,488,158 to Slaugh et al., entitled "Hydroformylation Of Olefins;" U.S. Patent No. 3,496,203 to Morris et al., entitled "Tertiary Organophosphine-Cobalt-Carbonyl Complexes;" U.S. Patent No. 3,496,204 to Morris et al., entitled "Tertiary Organophosphine-Cobalt-Carbonyl Complexes;" U.S. Patent No. 3,501,515 to Van Winkle et al., entitled "Bicyclic Heterocyclic Terteriary Phosphine-Cobalt-Carbonyl Complexes"; U.S. Patent No. 3,527,818 to Mason et al., entitled "Oxo Alcohols Using Catalysts Comprising Ditertiary Phosphines;" U.S. Patent Application Serial No. 10/075682, entitled "A Process For Preparing A Branched Olefin, A Method Of Using The Branched Olefin For Making A Surfactant, and a Surfactant " and in U. S. Patent Application Serial No. 10/167209 entitled "Process for the Preparation Of A Highly Linear Alcohol Composition." Methods of alcohol production are also described by Othmer, in "Encyclopedia of Chemical Technology" 2000, Fourth Edition; and by Wickson, in "Monohydric Alcohols; Manufacture, Applications and Chemistry" Ed. Am. Chem. Soc. 1981.

A hydroformylation catalyst used in hydroformylation unit 116 may include a metal from Group VIII of the Periodic Table. Examples of Groups VIII metals include cobalt, rhodium, nickel, palladium or platinum. The Group VIII metal may be used as a complex compound. A complex compound may be a Group VIII metal combined with a ligand. Examples of ligands include, but are not limited to, a phosphine, phosphite, arsine, stibine or pyridine ligand. Examples of hydroformylation catalysts include, but are not limited to, cobalt hydrocarbonyl catalyst, cobalt-phosphine ligand catalyst, rhodium-phosphine ligand catalyst or combinations thereof.

In hydroformylation unit 116, olefins may be hydroformylated using a continuous, semi-continuous or batch process. In case of a continuous mode of operation, the liquid hourly space velocities may be in the range of 0.1 h⁻¹ to 10 h⁻¹. When operating hydroformylation unit 116 as a batch process, reaction times may vary from 0.1 hours to 10 hours or even longer.

Reaction temperatures in hydroformylation unit 116 may range from 100 °C to 300 °C. In certain embodiments, reaction temperatures in the hydroformylation unit ranging from 125 °C to 250 °C may be used. Pressure in hydroformylation unit 116 may range from 1 atmosphere (101 kPa) to 300 atmospheres (30398 kPa). In an embodiment, a pressure from 20 (2027 kPa) to 150 atmospheres (15199 kPa) may be used. An amount of catalyst relative to the amount of olefin to be hydroformylated may vary. Typical molar ratios of catalyst to olefin in the hydrocarbon stream may range from 1:1000 to 10:1. A ratio of between 1:10 and 5:1 may be used in certain embodiments. In an embodiment, a second stream may be added to hydroformylation unit 116 to control reaction conditions. The second stream may include solvents that do not interfere substantially with the desired reaction. Examples of such solvents include, but are not limited to, alcohols, ethers, acetonitrile, sulfolane and paraffins.

Mono-alcohol selectivities of at least 90 percent and even of at least 92 percent may be achieved in hydroformylation unit 116. In addition, olefin conversions to aliphatic alcohols may range from 50 percent by weight to greater than 95 percent by weight. In certain embodiments, olefin conversion to aliphatic alcohols may be greater than 75 percent by weight. In some embodiments, olefin conversion to aliphatic alcohols may be greater than 99 percent by weight.

Isolation of aliphatic alcohols produced from the hydroformylation reaction product stream may be achieved by generally known methods. In an embodiment, isolation of the aliphatic alcohols includes subjecting the produced aliphatic alcohols to a first distillation, a saponification, a water washing treatment and a second distillation.

The hydroformylation reaction mixture stream may enter separator 126 via conduit 128. In separator 126, the hydroformylation reaction product stream may be subjected to a first distillation step (e.g., flash distillation or a short path distillation). In an embodiment, a short path distillation may be used to produce at least two streams, a bottom stream and a top stream. At least a portion of the bottom stream may be recycled to hydroformylation unit 116 via bottom stream recycle conduit 130, in certain embodiments. The top stream may include, but is not limited to, paraffins, unreacted olefins and a crude aliphatic alcohol product.

In an embodiment, a top stream may be subjected to a saponification treatment to remove any acids and esters present in the stream. Saponification may be performed by contacting the top stream with an aqueous solution of a hydroxide base (e.g., sodium hydroxide or potassium hydroxide) at elevated temperatures with agitation. The saponification may be carried out by contacting the top stream with an aqueous 0.5 percent to 10 percent hydroxide base solution at a crude alcohol/water ratio of 10:1 to 1:1 The amount of hydroxide base used may depend on an estimated amount of esters and acids present.

Saponification of the top stream may be carried out batch-wise or continuously: The top stream may be subjected to one or more saponification processes. Saponification reaction temperatures may be from 40 °C to 99 °C. In an embodiment, saponification temperatures may range from 60 °C to 95 °C. Mixing of the top stream with the basic water layer may be performed during the saponification reaction. Separation of the top stream from the basic water layer may be performed using known methods. The top stream may be subjected to a water wash after separation to remove any sodium salts present. The top stream may be separated using generally known techniques (e.g., fractional distillation) to produce at least two streams, a crude alcohol product stream and a paraffins and unreacted olefins stream. As used herein, "fractional distillation" refers to the distillation of liquids and subsequent collection of fractions of liquids determined by boiling point. The paraffins and unreacted olefins stream may be recycled, transported to other units for processing, stored on site, transported offsite and/or sold.

In certain embodiments, a crude aliphatic alcohol product stream may contain unwanted by-products (e.g., aldehydes, hemi-acetals). The by-products may be removed by subjecting the crude alcohol product stream to a hydrofinishing treatment step to produce an aliphatic alcohol product stream. "Hydrofinishing," as used herein, refers to a hydrogenation reaction carried out under relatively mild conditions. Hydrofinishing may be carried out using conventional hydrogenation processes. Conventional hydrogenation processes may include passing the crude alcohol feed together with a flow of hydrogen over a bed of a suitable hydrogenation catalyst. The aliphatic alcohol product stream may include greater than 50 percent by weight of the produced aliphatic alcohols. In some embodiments, the aliphatic alcohol product stream may include greater than 80 percent by weight of the produced aliphatic alcohols. In other embodiments, the aliphatic alcohol product stream may include greater than 95 percent by weight of the produced aliphatic alcohols. The aliphatic alcohol product stream may include branched aliphatic primary alcohols. The resulting aliphatic alcohols in the aliphatic alcohol product stream may be sold commercially, transported off-site, stored on site and/or used in other processing units via product conduit 132.

The composition of an aliphatic alcohol product stream may include hydrocarbons with an average carbon number ranging from 8 to 20. In an embodiment, an average carbon number of the hydrocarbons in aliphatic alcohol product stream may range from 10 to 18. The aliphatic alcohol product stream may include branched primary alcohols. The branched primary alcohol product may be suitable for the manufacture of anionic, nonionic and cationic surfactants. In some embodiments, branched primary alcohol products may be used as the precursor for the manufacture of anionic sulfates, including aliphatic sulfates and oxyalkyl sulfates and oxyalkyl alcohols.

Aliphatic alcohols may have slightly higher aliphatic branching and slightly higher number of quaternary carbons as the olefin precursor. In some embodiments, aliphatic branching may include methyl and/or ethyl branches. In other embodiments, aliphatic branching may include methyl, ethyl and higher aliphatic branching. In certain embodiments, a number of quaternary carbon atoms in the aliphatic alcohol product may be less than 0.5 percent. In other embodiments, a number of quaternary carbon atoms in the aliphatic alcohol product may be less than 0.3 percent. Branching of the alcohol product may be determined by ¹H NMR analysis. The number of quaternary carbon atoms may be determined by ¹³C NMR. A ¹³C NMR method for determining quaternary carbon atoms for branched aliphatic alcohols is described in U.S. Patent No. 6,150,322 to Singleton et al., entitled, "Highly Branched Primary Alcohol Compositions and Biodegradable Detergents Made Therefrom."

At least a portion of the paraffins and unreacted olefins stream may exit separation unit 126 and be transported via fourth conduit 134 to another processing unit, and/or storage vessel. At least a portion of the separated paraffins and unreacted olefins may enter dehydrogenation unit 136 via fourth conduit 134. An average carbon number of the hydrocarbons in the paraffins and unreacted olefins stream may range from 7 to 18. In certain embodiments, an average carbon number of the paraffins and unreacted olefins stream may range from 10 to 17. In some embodiments, an average carbon number of the paraffins and unreacted olefins stream may range from 10 to 13. In other embodiments, an average carbon number of the hydrocarbons in the paraffins and unreacted olefins stream may range from 14 to 17.

In an embodiment, at least a portion of the paraffins and unreacted olefins stream may be introduced into dehydrogenation unit 136 via fourth conduit 134. At least a portion of the unreacted paraffins in the hydrocarbon stream may be dehydrogenated to produce an olefinic hydrocarbon stream by use of a catalyst selected from a wide range of catalyst types. For example, the catalyst may be based on a metal and/or a metal compound deposited on a porous support. The metal or metal compound may include, but is not limited to, chrome oxide, iron oxide and noble metals.

Techniques of preparing catalysts, for performing the dehydrogenation step and for performing associated separation steps are generally known. For example, suitable procedures for preparing catalysts and performing the dehydrogenation step are described in U.S. Patent No. 5,012,021 to Vora et al., entitled "Process For the Production of Alkyl Aromatic Hydrocarbons Using Solid Catalysts;" U.S. Patent No. 3,274,287 to Moore et al., entitled "Hydrocarbon Conversion Process and Catalyst;" U.S. Patent No. 3,315,007 to Abell et al., entitled "Dehydrogenation of Saturated Hydrocarbons Over Noble-Metal Catalyst;" U.S. Patent No. 3,315,008 to Abell et al., entitled "Dehydrogenation of Saturated Hydrocarbons Over Noble-Metal Catalyst;" U.S. Patent No. 3,745,112 to Rausch, entitled "Platinum-Tin Uniformly Dispersed Hydrocarbon Conversion Catalyst and Process;" U.S. Patent No. 4,506,032 to Imai et al., entitled "Dehydrogenation Catalyst Composition" and U.S. Patent No. 4,430,517 to Imai et al., entitled "Dehydrogenation Process Using a Catalytic Composition."

Reaction conditions in dehydrogenation unit 136 may be varied to control unwanted side products (e.g., coke, dienes oligomers, cyclized hydrocarbons) and control double bond position in the olefin. In certain embodiments, temperatures may range from greater than 300 °C to less than 700 °C. In other embodiments, a dehydrogenation reaction temperature may range from 450 °C to 550 °C. During dehydrogenation, pressures in dehydrogenation unit 136 may range from greater than 0.010 atmosphere (1 kPa) to 25.0 atmospheres (2534 kPa). In an embodiment, a total pressure of dehydrogenation unit 136 during the reaction may range from 0.10 atmosphere (10 kPa) to 15.0 atmospheres (15200 kPa). In certain embodiments, pressure in dehydrogenation unit 136 may range from 1.0 atmosphere (101 kPa) to 5.0 atmospheres (510 kPa). In order to prevent coke from forming, hydrogen may be fed into dehydrogenation unit 136 together with the paraffins and unreacted olefins stream. The hydrogen to paraffins molar ratio may be set between 0.1 moles of hydrogen to 20 moles of paraffins. In some embodiments, a hydrogen to paraffin molar ratio is 1 to 10.

The amount of time (e.g., the residence time) that a process stream remains in dehydrogenation unit 136 may determine, to some extent, the amount of olefins produced. Generally, the longer a process stream remains in dehydrogenation unit 136, the conversion level of paraffins to olefins increases until an olefin-paraffin thermodynamic equilibrium is obtained. Residence time of the paraffins and unreacted olefins stream in dehydrogenation unit 136 may be such that the conversion level of paraffins to olefins may be kept below 50 mole percent. In certain embodiments, conversion level of paraffins to olefins may be kept in the range of from 5 to 30 mole percent. By keeping the conversion level low, side reactions may be prevented (e.g., diene formation and cyclization reactions).

In certain embodiments, at least a portion of non-converted paraffins may be separated from the olefinic stream and, if desired, the non-converted paraffins may be recycled to dehydrogenation unit 136 to undergo dehydrogenation. Such separation may be accomplished by extraction, distillation or adsorption techniques.

In some embodiments, at least a portion of a paraffinic hydrocarbon stream may be introduced upstream of dehydrogenation unit 136 to produce a combined stream. The combined stream may enter dehydrogenation unit 136 to undergo dehydrogenation. In other embodiments, a paraffinic hydrocarbon stream is introduced directly into dehydrogenation unit 136 through one or more points of entry.

The olefinic hydrocarbon stream may be combined with first hydrocarbon stream in first conduit 112 of isomerization unit 110 via fifth conduit 138. The combined stream may enter isomerization unit 110 and at least a portion of the olefins present in the combined stream may be isomerized to branched olefins. In some embodiments, an olefinic hydrocarbon stream may exit dehydrogenation unit 136 and be directly introduced into isomerization unit 110 through one or more points of entry.

In certain embodiments, additional hydrocarbon streams may be used to control reaction conditions and/or optimize the concentration of paraffins and unreacted olefins in isomerization unit 110, hydroformylation unit 116, dehydrogenation unit 136 and/or other processing units used to produce aliphatic alcohols. Referring to FIG. 3, a first hydrocarbon stream may be introduced into isomerization unit 110 via first conduit 112. The first hydrocarbon stream may include olefins and paraffins. Conditions of the olefin isomerization may be controlled, as previously described for System 100, such that the number of carbon atoms in the olefin prior to and subsequent to the isomerization conditions is substantially the same.

At least a portion of a paraffinic hydrocarbon stream may be introduced into first conduit 112 via sixth conduit 140 upstream of isomerization unit 110 to produce a combined stream. The combined stream may enter isomerization unit 110 via first conduit 112. In other embodiments, a paraffinic hydrocarbon stream is introduced directly into isomerization unit 110 through one or more points of entry.

At least a portion of the olefins in the combined stream may be isomerized to branched olefins in isomerization unit 110 to produce a second hydrocarbon stream. Addition of the paraffinic hydrocarbon stream may be used to optimize the olefin concentration in isomerization unit 110 and to control the extent of branching in the produced olefins. Concentration of paraffins in the paraffinic hydrocarbon stream may be between 10 percent and 99 percent by weight. In certain embodiments, a paraffin concentration may range between 10 percent and 50 percent by weight. In some embodiments, a paraffin concentration may range between 25 percent and 75 percent by weight. In other embodiments, a paraffinic stream may include olefins. An olefin concentration in the hydrocarbon stream may be between 20 and 80 percent.

The second hydrocarbon stream may exit isomerization unit 110 and be introduced into hydroformylation unit 116 via second conduit 114 to continue the process to produce aliphatic alcohols. The second hydrocarbon stream may include branched olefins. At least a portion of a third hydrocarbon stream may be introduced into second conduit 114 via seventh conduit 142 upstream of hydroformylation unit 116 to form a mixed stream. The mixed stream may be then introduced into hydroformylation unit 116 via second conduit 114. At least a portion of the olefins in the mixed stream may be hydroformylated using process conditions as previously described. In some embodiments, a third hydrocarbon stream may be introduced directly into hydroformylation unit 116 through one or more points of entry. It should be understood that an olefin concentration in the process streams may be adjusted by adding a stream through sixth conduit 140 only, seventh conduit 142 only, directly into hydroformylation unit 116 only or by combinations thereof.

The third hydrocarbon stream in conduit 142 may be used to optimize the olefin concentration in hydroformylation unit 116 to maximize hydroformylation of the olefins. The third hydrocarbon stream may be from the same source as the first hydrocarbon stream. Alternatively, the third hydrocarbon stream may be a hydrocarbon stream that includes olefins, paraffins, and/or hydrocarbon solvents derived from another source.

The third hydrocarbon stream may include olefins and paraffins. In certain embodiments, an average carbon number of the hydrocarbons in the third hydrocarbon stream ranges from 7 to 18. In certain embodiments, a third hydrocarbon stream may include olefins and paraffins. In some embodiments, a paraffin content of the third hydrocarbon stream may be between 60 percent and 90 percent by weight. In other embodiments, a paraffin content of the third hydrocarbon stream may be greater than 90 percent by weight.

In an embodiment, an olefin content of a third hydrocarbon stream ranges between 1 percent and 99 percent relative to the total hydrocarbon content. In certain embodiments, an olefin content of the third hydrocarbon stream may be between 45 percent and 99 percent by weight. In other embodiments, an olefin concentration of the third hydrocarbon stream may be greater than 80 percent by weight.

In some embodiments, a third hydrocarbon stream may include linear olefins. Addition of a stream that includes linear olefins downstream from the isomerization unit allows the creation of a hydroformylation feed stream that includes a mixture of linear and branched olefins. By introducing a stream including branched and linear olefins into hydroformylation unit 116 a mixture of branched and linear aliphatic alcohol products may be obtained. Varying the amount of linear olefins added to the hydroformylation feed stream may control the ratio of linear to branched aliphatic alcohol products. A mixture of branched and linear aliphatic alcohols may have improved properties when converted to surfactants or other products. Examples of improved surfactant properties include, but are not limited to, low skin and eye irritation, foaming properties, biodegradability, cold-water solubility and cold-water detergency. Applications for these surfactants include, but are not limited to, personal care products, household and industrial laundry products, hand dishwashing products, machine lubricant additives and lubricating oil formulations.

The hydroformylation reaction mixture stream may enter separator 126 via third conduit 128. Separation of the aliphatic alcohol product from at least a portion of the hydroformylation reaction stream may be performed in separation unit 126 as previously described. The separation may produce at least two streams, a bottom stream and a top stream using generally known techniques (e.g., distillation). At least a portion of the bottom stream may be recycled to hydroformylation unit 116 via recycle conduit 130. The top stream may be further purified and separated to produce at least two streams, a paraffins and unreacted olefins stream and a crude aliphatic alcohol product stream. At least a portion of the crude aliphatic alcohol product stream may be further purified to produce an aliphatic alcohol product stream using generally known techniques. The aliphatic alcohol product stream may exit separation unit 126 and be transported via product conduit 132 to be stored on site, sold commercially, transported off-site, and/or utilized in other processing units. Produced aliphatic alcohols in the aliphatic alcohol product stream may have an average carbon number from 8 to 19. In certain embodiments, produced aliphatic alcohols in the aliphatic alcohol product stream may have an average carbon number from 11 to 18. In some embodiments, produced aliphatic alcohols in the aliphatic alcohol product stream may have an average carbon number from 11 to 14. In other embodiments, produced aliphatic alcohols in the aliphatic alcohol product stream may have an average carbon number from 15 to 18.

At least a portion of the paraffins and unreacted olefins stream may exit separation unit 126 and be transported via fourth conduit 134 to another processing unit, and/or storage vessel. At least a portion of the separated paraffins and unreacted olefins may enter dehydrogenation unit 136 via fourth conduit 134. An average carbon number of the hydrocarbons in the paraffins and unreacted olefins stream may range from 7 to 18. In certain embodiments, an average carbon number of the paraffins and unreacted olefins stream may range from 10 to 17. In some embodiments, an average carbon number of the paraffins and unreacted olefins stream may range from 10 to 13. In other embodiments, an average carbon number of the hydrocarbons in the paraffins and unreacted olefins stream may range from 14 to 17.

At least a portion of the paraffins in the hydrocarbon stream may be dehydrogenated using process conditions as previously described. At least a portion of the resulting olefinic hydrocarbon stream may exit dehydrogenation unit 136 and be transported to another processing unit and/or a storage vessel via fifth conduit 138.

At least a portion of a paraffinic hydrocarbon stream may be introduced into fourth conduit 134 via eighth conduit 144 upstream of dehydrogenation unit 136 to produce a combined stream. The combined stream may enter dehydrogenation unit 136 via fourth conduit 134. In other embodiments, a paraffinic hydrocarbon stream is introduced directly into dehydrogenation unit 136 through one or more points of entry.

In certain embodiments, at least a portion of non-converted paraffins may be separated from dehydrogenated compounds in the olefinic stream. Such separation may be accomplished by extraction, distillation or, adsorption techniques. At least a portion of the non-converted paraffins may be recycled to dehydrogenation unit 136 to undergo further dehydrogenation.

At least a portion of the olefinic hydrocarbon stream may exit dehydrogenation unit 136 via fifth conduit 138 and be combined with the first hydrocarbon stream in first conduit 112 upstream of isomerization unit 110 to produce a combined stream. The combined stream may be introduced into isomerization unit 110 via first conduit 112 and at least a portion of the olefins in the combined stream may be isomerized to branched olefins. In some embodiments, an olefinic hydrocarbon stream may be introduced directly into isomerization unit 110 via one or more points of entry. Alternalively, at least a portion of the olefinic hydrocarbon stream may be combined with a second hydrocarbon stream in second conduit 114 downstream of isomerization unit 110 to produce a mixed stream. Depending on the dehydrogenation conditions, the mixed stream may include linear olefins. Addition of the olefinic hydrocarbon stream with the second hydrocarbon stream may produce a mixed stream that includes both linear and branched olefins.

Aliphatic alcohols may be converted to oxy alcohols, sulfates or other commercial products. At least a portion of the aliphatic alcohols in the alcohol product stream may be reacted in an oxyalkylation unit with an epoxide (e.g., ethylene oxide, propylene oxide, butylene oxide) in the presence of a base to produce an oxyalkyl alcohol. Condensation of an alcohol with an epoxide allows the alcohol functionality to be expanded by one or more oxy groups. The number of oxy groups may range from 3 to 12. For example, reaction of an alcohol with ethylene oxide may produce alcohol products having between 3 to 12 ethoxy groups. Reaction of an alcohol with ethylene oxide and propylene oxide may produce alcohols with an ethoxy/propoxy ratio of ethoxy to propoxy groups from 4:1 to 12:1. In some embodiments, a substantial proportion of alcohol moieties may become combined with more than three ethylene oxide moieties. In other embodiments, an approximately equal proportion may be combined with less than three ethylene oxide moieties. In a typical oxyalkylation product mixture, a minor proportion of unreacted alcohol may be present in the product mixture. In an embodiment, at least a portion of the aliphatic alcohol product stream may be formed by condensing a C₅ to C₃₁ aliphatic alcohol with an epoxide. In certain embodiments, a C₅ to C₁₅ branched primary alcohol may be condensed with ethylene oxide and/or propylene oxide. In other embodiments, a C₁₁ to C₁₇ branched primary alcohol may be condensed with ethylene oxide and/or propylene oxide. The resulting oxyalkyl alcohols may be sold commercially, transported off-site, stored on site and/or used in other processing units. In some embodiments, an oxyalkyl alcohol may be sulfated to form an anionic surfactant.

In an embodiment, at least a portion of the alcohols in the aliphatic alcohol product stream may be added to a base. The base may be an alkali metal or alkaline earth metal hydroxide (e.g., sodium hydroxide or potassium hydroxide). The base may act as a catalyst for the oxyalkylation reaction. An amount from 0.1 percent by weight to 0.6 percent by weight of a base, based on the total weight of alcohol, may be used for oxyalkylation of an alcohol. In an embodiment, a weight percent of a base may range from 0.1 percent by weight to 0.4 percent by weight based on the total alcohol amount. The reaction of the alcohol with the base may result in formation of an alkoxide. The resulting alkoxide may be dried to remove any water present. The dried alkoxide may be reacted with an epoxide. An amount of epoxide used may be from 1 mole to 12 moles of epoxide per mole of alkoxide. A resulting alkoxide-epoxide mixture may be allowed to react until the epoxide is consumed. A decrease in overall reaction pressure may indicate that the reaction is complete.

Reaction temperatures in an oxyalkylation unit may range from 120°C to 220 °C. In an embodiment, reaction temperatures may range from 140°C to 160 °C. Reaction pressures may be achieved by introducing to the reaction vessel the required amount of epoxide. Epoxides have a high vapor pressure at the desired reaction temperature. For consideration of process safety, the partial pressure of the epoxide reactant may be limited, for example, to less than 4 atmospheres (413 kPa). Other safety measures may include diluting the reactant with an inert gas such as nitrogen. For example, inert gas dilution may result in a vapor phase concentration of reactant of 50 percent or less. In some embodiments, an alcohol-epoxide reaction may be safely accomplished at a greater epoxide concentration, a greater total pressure and a greater partial pressure of epoxide if suitable, generally known, safety precautions are taken to manage the risks of explosion. With respect to ethylene oxide, a total pressure from 3 atmospheres (304 kPa) to 7 atmospheres (709 kPa) may be used. Total pressures of ethylene oxide from 1 atmosphere (101 kPa) to 4 atmospheres (415 kPa) may be used in certain embodiments. In an embodiment, total pressures from 1.5 atmospheres (150 kPa) to 3 atmospheres (304 kPa) with respect to ethylene oxide may be used. The pressure may serve as a measure of the degree of the reaction. The reaction may be considered substantially complete when the pressure no longer decreases with time.

Aliphatic alcohols and oxyalkyl alcohols may be derivatized to form compositions (e.g., sulfonates, sulfates, phosphates) useful in commercial product formulations (e.g., detergents, surfactants, oil additives, lubricating oil formulations). For example, alcohols may be sulfurized with SO₃ to produce sulfates. The term "sulfurized" refers to a sulfur atom or sulfur containing functionality being added to a carbon or oxygen. Sulfurization processes are described in U.S. Patent No. 6,462,215 to Jacobson et al., entitled "Sulfonation, Sulfation and Sulfamation"; U.S. Patent No. 6,448,435 to Jacobson et al., entitled "Sulfonation, Sulfation and Sulfamation"; U.S. Patent No. 3,462,525 to Levinsky et al, entitled, "Dental Compositions Comprising Long-Chain Olefin Sulfonates;" U.S. Pat. No. 3,428,654 to Rubinfeld et al., entitled, "Alkene Sulfonation Process and Products;" U.S. Patent No. 3,420,875 to DiSalvo et al., entitled, "Olefin Sulfonates;" U.S. Patent No. 3,506,580 to Rubinfeld et al., entitled, "Heat Treatment Of Sulfonated Olefin Products;" and U.S. Patent No. 3,579,537 to Rubinfeld, entitled, "Process For Separation Of Sultones From Alkenyl Sulfonic Acids."

A general class of aliphatic alcohol sulfates may be characterized by the chemical formula: (R-O-(A)ₓ-SO₃)ₙM. R' represents the aliphatic moiety. "A" represents a moiety of an alkylene oxide; x represents the average number of A moieties per R-O moiety and may range from 0 to 15; and n is a number depending on the valence of cation M. Examples of cation M include, but are not limited to, alkali metal ions, alkaline earth metal ions, ammonium ions and/or mixtures thereof. Examples of cations include, but are not limited to, magnesium, potassium, monoethanol amine, diethanol amine or triethanol amine.

Aliphatic and oxyalkyl alcohols may be sulfated in a sulfation unit. Sulfation procedures may include the reaction of sulfur trioxide (SO₃), chlorosulfonic acid (ClSO₃H), sulfamic acid (NH₂SO₃H) or sulfuric acid with an alcohol. In an embodiment, sulfur trioxide in concentrated (e.g., fuming) sulfuric acid may be used to sulfate alcohols. The concentrated sulfuric acid may have a concentration of 75 percent by weight to 100 percent by weight in water. In an embodiment, concentrated sulfuric acid may have a concentration of 85 percent by weight to 98 percent by weight in water. The amount of sulfur trioxide may range from 0.3 mole to 1.3 moles of sulfur trioxide per mole of alcohol. In certain embodiments, an amount of sulfur trioxide may range from 0.4 moles to 1.0 moles of sulfur trioxide per mole of alcohol.

In an embodiment, a sulfur trioxide sulfation procedure may include contacting a liquid alcohol or an oxyalkyl alcohol and gaseous sulfur trioxide in a falling film sulfator to produce a sulfuric acid ester of the alcohol. The reaction zone of the falling film sulfator may be operated at about atmospheric pressure and at a temperature in the range from 25 °C to 70 °C. The sulfuric acid ester of the alcohol may exit the falling film sulfator and enter a neutralization reactor. The sulfuric acid ester may be neutralized with an alkali metal solution to form the alkyl sulfate salt or the oxyalkyl sulfate salt. Examples of an alkali metal solution may include solutions of sodium or potassium hydroxide.

The derivatized alcohols may be used in a wide variety of applications. An example of an application includes detergent formulations. Detergent formulations include, but are not limited to, granular laundry detergent formulation, liquid laundry detergent formulations, liquid dishwashing detergent formulations and miscellaneous formulations. Examples of miscellaneous formulations may include general purpose cleaning agents, liquid soaps, shampoos and liquid scouring agents.

Granular laundry detergent formulations may include a number of components besides the derivatized alcohols (e.g., surfactants, builders, co-builders, bleaching agents, bleaching agent activators, foam controlling agents, enzymes, anti-graying agents, optical brighteners and stabilizers). Examples of other surfactants may include ionic, nonionic, amphoteric or cationic surfactants.

Liquid laundry detergent formulations may include the same components as granular laundry detergent formulations. In certain embodiments, liquid laundry detergent formulations may include less of an inorganic builder component than granular laundry detergent formulations. Hydrotropes may be present in the liquid detergent formulations. General purpose cleaning agents may include other surfactants, builders, foam control agents, hydrotropes and solubilizer alcohols.

The formulations may typically include one or more inert components. For example, the balance of liquid detergent formulations may typically be an inert solvent or diluent (e.g., water). Powdered or granular detergent formulations typically contain quantities of inert filler or carrier materials.

### EXAMPLES

### Example 1: Isomerization of Olefins in a Fischer-Tropsch derived Hydrocarbon

**Stream**. Carbon monoxide and hydrogen were reacted under Fischer-Tropsch process conditions to yield a hydrocarbon mixture of linear paraffins, linear olefms, a minor amount of dienes and a minor amount of oxygenates. The Fischer-Tropsch hydrocarbon stream was separated into different hydrocarbon streams using fractional distillation techniques. A hydrocarbon stream containing olefins and paraffins with an average number of carbon atoms from 8 to 10 was obtained. The composition of the resulting C₈-C₁₀ hydrocarbon stream was analysed by gas chromatography and is tabulated in Table 1.

**Table 1**

| **Fischer-Tropsch Hydrocarbon Stream Composition** | **Wt.%** |
|---|---|
| C₇ and lighter hydrocarbons | 0.12 |
| C₈ branched olefins | 0.02 |
| C₈ linear olefins | 0.75 |
| 1-Octene | 0.69 |
| n-Octane | 2.21 |
| C₉ branched olefins | 0.16 |
| C₉ linear olefins | 8.52 |
| 1-Nonene | 8.07 |
| n-Nonane | 20.03 |
| C₁₀ branched olefins | 0.28 |
| C₁₀ linear olefins | 22.92 |
| 1-Decene | 20.87 |
| n-Decane | 41.12 |
| C₁₁ and heavier hydrocarbons | 0.21 |
| C₉-C₁₁ alcohols | 3.56 |

A zeolite catalyst used for isomerization of linear olefins in the hydrocarbon stream was prepared in the following manner. Ammonium-ferrierite (645 grams) exhibiting a 5.4% loss on ignition and exhibiting the following properties: molar silica to alumina ratio of 62:1, surface area of 369 square meters per gram (P/Po=0.03), soda content of 480 ppm and n-hexane sorption capacity of 7.3 g per 100 g of ammonium-ferrierite was loaded into a Lancaster mix muller. CATAPAL® D alumina (91 grams) exhibiting a loss on ignition of 25.7% was added to the muller. During a five-minute mulling period, 152 milliliters of deionized water was added to the alumina/ammonium-ferrierite mixture. Next, a mixture of 6.8 grams glacial acetic acid, 7.0 grams of citric acid and 152 milliliters of deionized water was slowly added to the alumina/ammonium-ferrierite mixture in the muller to peptize the alumina. The resulting alumina/ammoniurn-ferrierite/acid mixture was mulled for 10 minutes. Over a period of 15 minutes, a mixture of 0.20 grams of tetraamine palladium nitrate in 153 grams of deionized water was slowly added to mulled alumina/ammonium-fernerite/acid mixture. The resulting mixture exhibited a 90:10 ratio of zeolite to alumina and a loss on ignition of 43.5%. The zeolite/alumina mixture was shaped by extruding the mixture through a stainless steel die plate (1.59 mm (1/16") holes) of a 5.72 cm (2.25) inch Bonnot extruder. The moist zeolite/alumina extrudate was dried at 125°C for 16 hours. After drying, the zeolite/alumina extrudate was longsbroken manually. The zeolite/alumina extrudate was calcined in flowing air at 200°C for two hours. The temperature was raised to a maximum temperature of 500°C and the zeolite/alumina extrudate was calcined for an additional two hours to yield an isomerization catalyst. The isomerization catalyst was allowed to cool in a dessicator under a nitrogen atmosphere.

Stainless steel tubing, 2.54 cm (1 inch) OD, 1.52 cm (0.6 inch) ID and 66 cm (26 inches) long, was used as an isomerization reactor. A thermowell extended 50.8 cm (20 inches) from the top of the stainless steel reactor tube. To load the reactor tube, the reactor tube was inverted and a piece of glass wool was transferred down the wall of the reactor tube, over the thermowell and positioned at the bottom of the reactor tube to serve as a plug for the reactor tube. Silicon carbide (20 mesh) was added to a depth of about 15.2 cm (6 inches) to the reactor tube. A second piece of glass wool was placed over the silicon carbide. A mixture of 6.0 grams of the isomerization catalyst particles (6-20 mesh) and 45 grams of fresh silicon carbide (60-80 mesh) was added to the reactor tube in two parts. The two-part addition distributed the isomerization catalyst evenly in the reactor tube and resulted in an isomerization catalyst bed of about 25.4 cm (10 inches) in length. A third piece of glass wool was added to the top of the catalyst in the reactor tube. Silicon carbide (20 mesh) was layered onto the third piece of glass wool. A fourth piece of glass wool was positioned over the silicon carbide to serve as a plug for the bottom of the reactor tube. To monitor the temperature of the reaction at various points in the reactor tube, a multipoint thermocouple was inserted into the thermowell of the reactor tube. The temperature above, below and at three different places in the catalyst bed was monitored. The reactor tube was inverted and installed in the furnace. The reactor tube was heated to the operating temperature of 280 °C over a four-hour period under flowing nitrogen. Once the temperature of 280 °C was obtained, the reactor tube was held at the operating temperature for an additional two hours to condition the isomerization catalyst.

After conditioning the isomerization catalyst, the hydrocarbon stream was pumped through the reactor tube at a flow rate of 60 g/hr. Nitrogen, at a flow rate of 6 L/hr, was passed over the isomerization catalyst simultaneously with the hydrocarbon stream. The hydrocarbon stream was vaporized before contacting the isomerization catalyst. The reactor tube was operated at an outlet pressure of 20 kPa above atmospheric pressure.

In Table 2, the weight percent of C₈-C₁₀ branched olefins, C₈-C₁₀ linear olefins and C₈-C₁₀ paraffins in the hydrocarbon stream at 0 hours and in the reactor tube effluent after 24 and 48 hours of isomerization is tabulated. Greater than 90% of the linear olefins in the hydrocarbon stream were converted into branched olefins in the isomerization reactor. During the isomerization step, a small amount of material boiling below C₈ was generated from cracking side reactions. In addition, a portion of the C₉-C₁₁ alcohols present in the feed was dehydrated to yield additional olefins in the product. The average number of alkyl branches on the C₈-C₁₀ olefins in the product was found to be 1.0 as determined by ¹H NMR analysis.

**Table 2**

| **Fischer-Tropsch Hydrocarbon Stream Composition During Isomerization Reaction** | **0 Hr Wt.%** | **24 Hr Wt.%** | **48 Hr Wt.%** |
|---|---|---|---|
| C₈-C₁₀ branched olefins | 0.46 | 33.04 | 33.16 |
| C₈-C₁₀ linear olefins | 32.19 | 2.52 | 2.54 |
| C₈-C₁₀ paraffins | 63.19 | 63.32 | 63.27 |
| Branched to linear C₈-₁₀ olefin ratio | 0.1 | 13.1 | 13.1 |

### Example 2. Isomerization of 1-Dodecene: 1-dodecene was obtained from Shell Chemical Co. The composition of 1-dodecene, as assayed by gas chromatography, is tabulated in Table 3.

**Table 3**

| **1-Dodecene Composition** | **Wt.%** |
|---|---|
| 1-Dodecene | 98.0 |
| Other C₁₀-C₁₄ olefins | 1.2 |
| <C₁₀ hydrocarbons | 0.2 |
| >C₁₄ hydrocarbons | 0.2 |
| Paraffins | 0.4 |
| Total C₁₀-C₁₄ hydrocarbons | 99.6 |

1-dodecene was isomerized using the same reactor tube design and isomerization catalyst preparation as described in Example 1. A stream of 1-dodecene was pumped through a reactor tube at a flow rate of 90 g/hr. Nitrogen, at a flow rate of 6 L/hr, was passed over the isomerization catalyst simultaneously with the stream of 1-dodecene. The stream of 1-dodecene was vaporised before contacting the isomerization catalyst. The reactor tube was operated at an outlet pressure of 20 kPa above atmospheric pressure and a temperature of 290°C.

Table 4 is a tabulation of the weight percent of less than C₁₀, C₁₀-C₁₄ and greater than C₁₄ molecules in 1-dodecene at 0 hours and the reactor tube effluent after 168 and 849 hours. Linear C₁₀-C₁₄ olefins were converted in a 94% yield to branched C₁₀-C₁₄ olefins after a 168 hr processing time. During the isomerization step, less than 3 weight percent of material boiling below C₁₀ was generated from cracking side reactions. The average number of alkyl branches on the C₁₀-C₁₄ olefins in the product was determined to be 1.3 by ¹H NMR analysis.

**Table 4**

| **1-Dodecene Stream Composition During Isomerization Reaction** | **0 Hr Wt.%** | **168 Hr Wt.%** | **849 Hr Wt.%** |
|---|---|---|---|
| <C₁₀ hydrocarbons | 0.2 | 2.5 | 2.4 |
| C₁₀-C₁₄ hydrocarbons | 99.6 | 97.2 | 97.4 |
| >C₁₄ hydrocarbons | 0.2 | 0.3 | 0.2 |
| Branched C₁₀-C₁₄ olefins | 0.6 | 93.2 | 93.4 |
| Linear C₁₀-C₁₄ olefins | 99.0 | 2.8 | 2.9 |
| Paraffins | 1.0 | 2.0 | 1.9 |

### Example 3. Dehydrogenation of Dodecane with Minimal Isomerization: Dodecane was obtained from Aldrich Chemical Company and stored under nitrogen before being processed. The composition of dodecane, as assayed by gas chromatography, is tabulated in Table 5.

**Table 5**

| **Dodecane Composition** | **Wt.%** |
|---|---|
| Dodecane | 99.3 |
| <C₁₀ hydrocarbons | <0.1 |
| C₁₀, C₁₁, C₁₃ and C₁₄ hydrocarbons | <0.6 |
| >C₁₄ hydrocarbons | <0.1 |
| Other C₁₀-C₁₄ olefins | <0.1 |

A paraffin dehydrogenation catalyst was prepared according to Example 1 (catalyst A) of U.S. Patent No. 4,430,517 to Imai et al., entitled "Dehydrogenation Process Using A Catalytic Composition." The resulting catalyst included 0.8 wt.% platinum, 0.5 wt.% tin, 2.7 wt.% tin, 2.7 wt.% potassium and 1.3 wt.% chlorine on a gamma-alumina support. The atomic ratio of potassium to platinum for this catalyst was 16.8.

The dehydrogenation catalyst was prepared by dissolving substantially pure aluminum pellets in a hydrochloric acid solution. An amount of stannic chloride was added to the resulting solution to provide a final composite containing 0.5 weight % tin and stirred to distribute the tin component evenly throughout the mixture. Hexamethylenetetramine was added to the resulting tin mixture and the resulting tin-amine mixture was dropped into an oil bath in a manner to form spherical particles having an average particle diameter of about 1.59 mm (1/16 inch). The spheres were aged, washed with an ammoniacal solution, dried and calcined to form a spherical gamma-alumina carrier material. The resulting spheres contained about 0.5 weight % tin in the form of tin oxide. More details about the method of preparing the alumina carrier material are disclosed in U.S. Patent No. 2,620,314 to Hoesktra, entitled, "Spheroidal Alumina."

The tin-alumina composite was contacted with a deionized solution of chloroplatinic acid and hydrochloric acid (2 weight percent based on alumina weight) in a rotary drier for 15 minutes at room temperature. The amount of chloroplatinic acid used was the amount necessary to incorporate 0.8 weight percent platinum into the tin-alumina composite. The solution was then heated and purged with nitrogen to remove water resulting in a platinum-chlorine-tin-alumina composite. The incorporated chlorine was removed by heating the platinum-chlorine-tin-alumina composite to 550 °C and treating the composite with a 50/50 air/80 °C steam mixture at a gas hourly space velocity (GHSV) of 300 hr⁻¹. After treatment with the air/steam mixture, the platinum-tin-alumina composite contained less than 0.1 weight percent chlorine.

The platinum-tin-alumina composite was contacted with a deionized water solution of potassium nitrate. The amount of potassium nitrate used was the amount necessary to incorporate 2.7 weight percent of potassium in the platinum-tin-alumina composite. The water was removed from the platinum-tin-potassium-alumina composite by heating the composite to 100 °C under a purge of dry air (1000 hr⁻¹GHSV for 0.5 hour. The temperature was raised to 525°C and the platinum-tin-potassium alumina composite was treated with a stream of hydrochloric acid (12 cc/hr, 0.9 M HCl) and a stream of 50/50 air/80 °C steam mixture (300 hr⁻¹ GHSV) to incorporate chlorine into the platinum-tin-potassium-alumina composite. The platinum-tin-potassium-chlorine-alumina composite was dried at 525 °C under a purge of dry air (1000 hr⁻¹ GHSV). The resulting catalyst spheres had an average particle diameter of 1.59 mm (1/16 inch) and were crushed and sized into 6-20 mesh particle before testing.

Stainless steel tubing, 2.54 cm (1 inch) OD, 1.62 cm (0.6 inch) ID and 66 cm (26 inches) long, was used as an isomerization reactor. A thermowell extended 50.8 cm (20 inches) from the top of the stainless steel reactor tube. To load the reactor tube, the reactor tube was inverted and a piece of glass wool was transferred down the wall of the reactor tube, over the thermowell and positioned at the bottom of the reactor tube to serve as a plug for the reactor tube. Silicon carbide (20 mesh) was added to a depth of about 15.2 cm (6 inches) to the reactor tube. A second piece of glass wool was placed over the silicon carbide. A mixture of 6.0 grams of platinum-tin on alumina catalyst particles (6-20 mesh) and 45 grams of fresh silicon carbide (60-80 mesh) was added to the reactor tube in two parts. The two-part addition distributed the catalyst evenly in the reactor tube and resulted in a catalyst bed of about 25.4 cm (10 inches) in length. A third piece of glass wool was added to the top of the catalyst in the reactor tube. Silicon carbide (20 mesh) was layered onto the third piece of glass wool. A fourth piece of glass wool was positioned over the silicon carbide to serve as a plug for the bottom of the reactor tube. To monitor the temperature of the reaction at various points in the reactor tube, a multipoint thermocouple was inserted into the thermowell of the reactor tube. The temperature above, below and at three different places in the catalyst bed was monitored. The reactor tube was inverted and installed in the furnace. The reactor tube was purged with nitrogen. The reactor tube was heated to the operating temperature of 425°C over a four-hour period under flowing nitrogen (250 standard liters per hour). Once the temperature of 425°C was obtained, the reactor tube was held at the operating temperature for an additional two hours. The catalyst was presulfided by flowing a 1% mixture of hydrogen sulfide gas in hydrogen gas at 425 °C for five minutes through the reactor tube. After 5 minutes, the hydrogen sulfide in hydrogen gas flow was switched to a hydrogen gas flow through the reactor tube.

After presulfiding the catalyst, the reactor tube was maintained at 425°C for eight hours. After eight hours, the reactor tube pressure was increase to 274 kPa (25 psig) with hydrogen gas. Dodecane was pumped through the reactor tube at a flow rate of 40 g/hr at a hydrogen flow rate of 125 standard liters per hour. After four hours, the dodecane stream was increased to 80 g/hr. After obtaining a flow rate of 80 g/hr, the reactor tube temperature was raised to 460°C. The reactor tube was sampled every eight hours after obtaining the operating temperature of 460°C.

After twenty-four hours the weight percent of dodecane was 11.4 weight percent as depicted in Table 6. At a temperature of 479 °C, the conversion of dodecane to olefins was 16 weight percent after twenty-four hours. Of the olefins, formed 84 weight percent were mono olefins, 4.1 weight percent were aromatic compounds and 7.5 weight percent were di-olefins. Of the total amount of olefins formed, 6 percent were branched, as determined by ¹H NMR analysis.

**Table 6**

| **Test Results.** | |
|---|---|
| Conversion (wt.%) after 24 hours on-stream at 460°C. | 11.4 |
| Temperature required for 16 wt. % conversion | 479°C |
| Selectivity to mono olefins at 16 wt. % conversion. | 84 wt.% |
| Selectivity to aromatics at 16 wt. % conversion. | 4.1 wt.% |
| Selectivity to di-olefins at 16 wt. % conversion. | 7.5 wt.% |
| % Branched C₁₂ olefins in total C₁₂ olefins | 6 |

## Claims

1. A method for the production of aliphatic alcohols, comprising:
introducing a first hydrocarbon stream comprising linear olefins and paraffins into an isomerization unit, wherein the isomerization unit is configured to isomerize at least a portion of linear olefins in the first hydrocarbon stream to branched olefins,
isomerizing at least a portion of the linear olefins in the first hydrocarbon stream to branched olefins in the isomerization unit, wherein at least a portion of the unreacted components of the first hydrocarbon stream and at least a portion of the produced branched olefins form a second hydrocarbon stream;
introducing at least a portion of the second hydrocarbon stream into a hydroformylation unit, wherein the hydroformylation unit is configured to hydroformylate at least a portion of the olefins in the second hydrocarbon stream to produce aliphatic alcohols, and wherein at least a portion of the produced aliphatic alcohols comprises a branched alkyl group, and wherein at least a portion of the unreacted components of the second hydrocarbon stream, and at least a portion of the produced aliphatic alcohols form a hydroformylation reaction stream;
separating at least a portion of the hydroformylation reaction stream to produce an aliphatic alcohol product stream and a paraffins and unreacted olefins stream; and
introducing at least a portion of the paraffins and unreacted olefins stream into a dehydrogenation unit, wherein the dehydrogenation unit is configured to
dehydrogenate at least a portion of paraffins in the paraffins and unreacted olefins stream to produce olefins, and wherein at least a portion of the produced olefins exits the dehydrogenation unit to form an olefinic hydrocarbon stream; and
introducing at least a portion of the olefinic hydrocarbon stream into the isomerization unit.

2. The method of claim 1, wherein the first hydrocarbon stream is produced from an olefin oligomerization process.

3. The method of claim 1, wherein the first hydrocarbon stream is produced from a Fischer-Tropsch process.

4. The method of any one of claims 1 to 3, wherein the first hydrocarbon stream comprises olefins and paraffins having a carbon number from 10 to 17.

5. The method of any one of claims 1 to 4, wherein the isomerization unit is operated at a reaction temperature between 200°C and 500 °C.

6. The method of any one of claims 1 to 5, wherein the isomerization unit is operated at hydrocarbon partial pressure ranging from 10 kPa (0.1 atmosphere) to 2026 kPa (20 atmospheres).

7. The method of any one of claims 1 to 6 wherein the hydroformylation unit is operated at a reaction temperature from 100°C to 300 °C.

8. The method of any one of claims 1 to 7, further comprising adjusting a ratio of olefins to paraffins introduced into the isomerization unit by adding at least a portion of a paraffinic hydrocarbon stream into the isomerization unit.

9. The method of any one of claims 1 to 8, further comprising:
adjusting a ratio of olefins to paraffins introduced into the isomerization unit by combining a paraffinic hydrocarbon stream with at least a portion of the first hydrocarbon stream upstream of the isomerization unit to form a combined stream; and,
introducing the combined stream into the isomerization unit.

10. The method of any one of claims 1 to 9, further comprising:
adjusting a ratio of olefins to paraffins introduced into the isomerization unit by combining at least a portion of a paraffinic hydrocarbon stream with at least a portion of the first hydrocarbon stream upstream of the isomerization unit to form a combined stream;
introducing the combined stream into the isomerization unit;
adjusting a ratio of olefins to paraffins introduced into the hydroformylation unit by combining at least a portion of a third hydrocarbon stream with at least a portion of the second hydrocarbon stream upstream of the hydroformylation unit to form a combined stream; and,
introducing the combined stream into the hydroformylation unit.

11. The method of any one of claims 1 to 10, further comprising adjusting a ratio of olefins to paraffins introduced into the hydroformylation unit by adding at least a portion of a third hydrocarbon stream into the hydroformylation unit.

12. The method of any one of claims 1 to 10, further comprising adjusting a ratio of olefins to paraffins introduced into the hydroformylation unit by adding at least a portion of a third hydrocarbon stream into the hydroformylation unit, wherein the third hydrocarbon stream comprises greater than 80 percent olefins by weight.

13. The method of any one of claims 1 to 10, further comprising:
adjusting a ratio of olefins to paraffins introduced into the hydroformylation unit by combining at least a portion of a third hydrocarbon stream with at least a portion of the second hydrocarbon stream upstream of the hydroformylation unit to form a combined stream; and,
introducing the combined stream into the hydroformylation unit.

14. The method of any one of claims 1 to 10 further comprising:
adjusting a ratio of olefins to paraffins introduced into the hydroformylation unit by combining at least a portion of a third hydrocarbon stream with at least a portion of the second hydrocarbon stream upstream of the hydroformylation unit to form a combined stream, wherein the third hydrocarbon stream comprises greater than 80 percent olefins by weight; and,
introducing the combined stream into the hydroformylation unit.

15. The method of any one of claims 1 to 10, further comprising:
adjusting a ratio of olefins to paraffins introduced into the hydroformylation unit by combining at least a portion of a third hydrocarbon stream with at least a portion of the second hydrocarbon stream upstream of the hydroformylation unit to form a combined stream, wherein the third hydrocarbon stream comprises linear olefins; and,
introducing the combined stream into the hydroformylation unit.

16. The method of any one of claims 1 to 10, further comprising:
adjusting a ratio of olefins to paraffins introduced into the hydroformylation unit by combining at least a portion of a third hydrocarbon stream with at least a portion of the second hydrocarbon stream upstream of the hydroformylation unit to form a combined stream, wherein at least a portion of the second hydrocarbon stream comprises branched olefins; and,
introducing the combined stream into the hydroformylation unit.

17. The method of any one of claims 1 to 10, further comprising:
adjusting a ratio of olefins to paraffins introduced into the hydroformylation unit by combining at least a portion of a third hydrocarbon stream with at least a portion of the second hydrocarbon stream upstream of the hydroformyladon unit to form a combined stream, wherein at least a portion of the third hydrocarbon stream comprises linear olefins and at least a portion of the second hydrocarbon stream comprises branched olefins; and,
introducing the combined stream into the hydroformylation unit.

18. The method of any one of claims 1 to 17, wherein the dehydrogenation unit is operated at a temperature between 300°C and 700 °C.

19. The method of any one of claims 1 to 18, wherein the dehydrogenation unit is operated at a pressure between 1 kPa (0.01 atmosphere) and 2534 kPa (25 atmospheres).

20. The method of any one of claims 1 to 19, further comprising introducing at least a portion of the aliphatic alcohol product stream into a sulfation unit, wherein the sulfation unit is configured to sulfate at least a portion of the aliphatic alcohols in the aliphatic alcohol product stream to produce aliphatic sulfates, and wherein at least a portion of the aliphatic sulfates produced comprises branched aliphatic sulfates.

21. The method of any one of claims 1 to 19, further comprising introducing at least a portion of the aliphatic alcohol product stream into an oxyalkylation unit, wherein the oxyalkylation unit is configured to oxyalkylate at least a portion of the aliphatic alcohols in the aliphatic alcohol product stream to produce oxyalkyl alcohols, wherein at least a portion of the oxyalkyl alcohols produced comprises branched oxyalkyl alcohols.

## Patentansprüche

1. Verfahren zur Herstellung von aliphatischen Alkoholen, umfassend:
Einbringen eines ersten Kohlenwasserstoffstroms, der lineare Olefine und Paraffine umfasst, in eine Isomerisationseinheit, wobei die Isomerisationseinheit so ausgebildet ist, dass mindestens ein Teil der linearen Olefine im ersten Kohlenwasserstoffstrom zu verzweigten Olefinen isomerisiert wird,
Isomerisieren von mindestens einem Teil der linearen Olefine im ersten Kohlenwasserstoffstrom zu verzweigten Olefinen in der Isomerisationseinheit, wobei mindestens ein Teil der nicht umgesetzten Bestandteile des ersten Kohlenwasserstoffstroms und mindestens ein Teil der hergestellten verzweigten Olefine einen zweiten Kohlenwasserstoffstrom bilden;
Einbringen von mindestens einem Teil des zweiten Kohlenwasserstoffstroms in eine Hydroformylierungseinheit, wobei die Hydroformylierungseinheit so ausgebildet ist, dass mindestens ein Teil der Olefine im zweiten Kohlenwasserstoffstrom zur Herstellung von aliphatischen Alkoholen hydroformyliert wird, und wobei mindestens ein Teil der hergestellten aliphatischen Alkohole eine verzweigte Alkylgruppe umfasst und mindestens ein Teil der nicht umgesetzten Bestandteile des zweiten Kohlenwasserstoffstroms und mindestens ein Teil der hergestellten aliphatischen Alkohole einen Hydroformylierungsreaktionsstrom bilden;
Trennen von mindestens einem Teil des Hydroformylierungsreaktionsstroms zur Herstellung eines Produktstroms aus aliphatischen Alkoholen und eines Stroms aus Paraffinen und nicht umgesetzten Olefinen; und
Einbringen von mindestens einem Teil des Stroms aus Paraffinen und nicht umgesetzten Olefinen in eine Dehydrierungseinheit, wobei die Dehydrierungseinheit so ausgebildet ist, dass mindestens ein Teil der Paraffine in dem Strom aus Paraffinen und nicht umgesetzten Olefinen zur Herstellung von Olefinen dehydriert wird, und wobei mindestens ein Teil der hergestellten Olefine aus der Dehydrierungseinheit austritt, um einen olefinischen Kohlenwasserstoffstrom zu bilden; und
Einbringen von mindestens einem Teil des olefinischen Kohlenwasserstoffstroms in die Isomerisationseinheit.

2. Verfahren nach Anspruch 1, wobei der erste Kohlenwasserstoffstrom aus einem Olefinoligomerisierungsverfahren hergestellt wird.

3. Verfahren nach Anspruch 1, wobei der erste Kohlenwasserstoffstrom durch ein Fischer-Tropsch-Verfahren hergestellt wird.

4. Verfahren nach einem der Ansprüche 1 bis 3, wobei der erste Kohlenwasserstoffstrom Olefine und Paraffine mit einer Kohlenstoffzahl von 10 bis 17 umfasst.

5. Verfahren nach einem der Ansprüche 1 bis 4, wobei die Isomerisationseinheit bei einer Reaktionstemperatur zwischen 200°C und 500°C betrieben wird.

6. Verfahren nach einem der Ansprüche 1 bis 5, wobei die Isomerisationseinheit bei einem
Kohlenwasserstoffpartialdruck im Bereich von 10 kPa (0,1 Atmosphären) bis 2026 kPa (20 Atmosphären) betrieben wird.

7. Verfahren nach einem der Ansprüche 1 bis 6, wobei die Hydroformylierungseinheit bei einer Reaktionstemperatur von 100°C bis 300°C betrieben wird.

8. Verfahren nach einem der Ansprüche 1 bis 7, weiter umfassend das Einstellen eines Verhältnisses von in die Isomerisationseinheit eingebrachten Olefinen zu Paraffinen durch Zugabe von mindestens einem Teil eines paraffinischen Kohlenwasserstoffstroms in die Isomerisationseinheit.

9. Verfahren nach einem der Ansprüche 1 bis 8, weiter umfassend:
Einstellen eines Verhältnisses von in die Isomerisationseinheit eingebrachten Olefinen zu Paraffinen durch Kombination eines paraffinischen Kohlenwasserstoffstroms mit mindestens einem Teil des ersten Kohlenwasserstoffstroms vor der Isomerisationseinheit zur Bildung eines kombinierten Stroms; und
Einbringen des kombinierten Stroms in die Isomerisationseinheit.

10. Verfahren nach einem der Ansprüche 1 bis 9, weiter umfassend:
Einstellen eines Verhältnisses von in die Isomerisationseinheit eingebrachten Olefinen zu Paraffinen durch Kombination von mindestens einem Teil eines paraffinischen Kohlenwasserstoffstroms mit mindestens einem Teil des ersten Kohlenwasserstoffstroms vor der Isomerisationseinheit zur Bildung eines kombinierten Stroms;
Einbringen des kombinierten Stroms in die Isomerisationseinheit;
Einstellen eines Verhältnisses von in die Hydroformylierungseinheit eingebrachten Olefinen zu Paraffinen durch Kombination von mindestens einem Teil eines dritten Kohlenwasserstoffstroms mit mindestens einem Teil des zweiten Kohlenwasserstoffstroms vor der Hydroformylierungseinheit zur Bildung eines kombinierten Stroms; und
Einbringen des kombinierten Stroms in die Hydroformylierungseinheit.

11. Verfahren nach einem der Ansprüche 1 bis 10, weiter umfassend das Einstellen eines Verhältnisses von in die Hydroformylierungseinheit eingebrachten Olefinen zu Paraffinen durch Zugabe von mindestens einem Teil eines dritten Kohlenwasserstoffstroms in die Hydroformylierungseinheit.

12. Verfahren nach einem der Ansprüche 1 bis 10, weiter umfassend das Einstellen eines Verhältnisses von in die Hydroformylierungseinheit eingebrachten Olefinen zu Paraffinen durch Zugabe von mindestens einem Teil eines dritten Kohlenwasserstoffstroms in die Hydroformylierungseinheit, wobei der dritte Kohlenwasserstoffstrom mehr als 80 Gew.-% Olefine umfasst.

13. Verfahren nach einem der Ansprüche 1 bis 10, weiter umfassend:
Einstellen eines Verhältnisses von in die Hydroformylierungseinheit eingebrachten Olefinen zu Paraffinen durch Kombination von mindestens einem Teil eines dritten Kohlenwasserstoffstroms mit mindestens einem Teil des zweiten Kohlenwasserstoffstroms vor der Hydroformylierungseinheit zur Bildung eines kombinierten Stroms; und
Einbringen des kombinierten Stroms in die Hydroformylierungseinheit.

14. Verfahren nach einem der Ansprüche 1 bis 10, weiter umfassend:
Einstellen eines Verhältnisses von in die Hydroformylierungseinheit eingebrachten Olefinen zu Paraffinen durch Kombination von mindestens einem Teil eines dritten Kohlenwasserstoffstroms mit mindestens einem Teil des zweiten Kohlenwasserstoffstroms vor der Hydroformylierungseinheit zur Bildung eines kombinierten Stroms, wobei der dritte Kohlenwasserstoffstrom mehr als 80 Gew.-% Olefine umfasst; und
Einbringen des kombinierten Stroms in die Hydroformylierungseinheit.

15. Verfahren nach einem der Ansprüche 1 bis 10, weiter umfassend:
Einstellen eines Verhältnisses von in die Hydroformylierungseinheit eingebrachten Olefinen zu Paraffinen durch Kombination von mindestens einem Teil eines dritten Kohlenwasserstoffstroms mit mindestens einem Teil des zweiten Kohlenwasserstoffstroms vor der Hydroformylierungseinheit zur Bildung eines kombinierten Stroms, wobei der dritte Kohlenwasserstoffstrom lineare Olefine umfasst; und
Einbringen des kombinierten Stroms in die Hydroformylierungseinheit.

16. Verfahren nach einem der Ansprüche 1 bis 10, weiter umfassend:
Einstellen eines Verhältnisses von in die Hydroformylierungseinheit eingebrachten Olefinen zu Paraffinen durch Kombination von mindestens einem Teil eines dritten Kohlenwasserstoffstroms mit mindestens einem Teil des zweiten Kohlenwasserstoffstroms vor der Hydroformylierungseinheit zur Bildung eines kombinierten Stroms, wobei mindestens ein Teil des zweiten Kohlenwasserstoffstroms verzweigte Olefine umfasst; und
Einbringen des kombinierten Stroms in die Hydroformylierungseinheit.

17. Verfahren nach einem der Ansprüche 1 bis 10, weiter umfassend:
Einstellen eines Verhältnisses von in die Hydroformylierungseinheit eingebrachten Olefinen zu Paraffinen durch Kombination von mindestens einem Teil eines dritten Kohlenwasserstoffstroms mit mindestens einem Teil des zweiten Kohlenwasserstoffstroms vor der Hydroformylierungseinheit zur Bildung eines kombinierten Stroms, wobei mindestens ein Teil des dritten Kohlenwasserstoffstroms lineare Olefine umfasst und mindestens ein Teil des zweiten Kohlenwasserstoffstroms verzweigte Olefine umfasst; und
Einbringen des kombinierten Stroms in die Hydroformylierungseinheit.

18. Verfahren nach einem der Ansprüche 1 bis 17, wobei die Dehydrierungseinheit bei einer Temperatur zwischen 300°C und 700°C betrieben wird.

19. Verfahren nach einem der Ansprüche 1 bis 18, wobei die Dehydrierungseinheit bei einem Druck zwischen 1 kPa (0,01 Atmosphären) und 2534 kPa (25 Atmosphären) betrieben wird.

20. Verfahren nach einem der Ansprüche 1 bis 19, weiter umfassend das Einbringen von mindestens einem Teil des Produktstroms aus aliphatischen Alkoholen in eine Sulfatierungseinheit, wobei die Sulfatierungseinheit so ausgebildet ist, dass mindestens ein Teil der aliphatischen Alkohole in dem Produktstrom aus aliphatischen Alkoholen zur Herstellung von aliphatischen Sulfaten sulfatiert wird, und wobei mindestens ein Teil der hergestellten aliphatischen Sulfate verzweigte aliphatische Sulfate umfasst.

21. Verfahren nach einem der Ansprüche 1 bis 19, weiter umfassend das Einbringen von mindestens einem Teil des Produktstroms aus aliphatischen Alkoholen in eine Oxyalkylierungseinheit, wobei die Oxyalkylierungseinheit so ausgebildet ist, dass mindestens ein Teil der aliphatischen Alkohole in dem Produktstrom aus aliphatischen Alkoholen zur Herstellung von Oxyalkylalkoholen oxyalkyliert wird, wobei mindestens ein Teil der hergestellten Oxyalkylalkohole verzweigte Oxyalkylalkohole umfasst.

## Revendications

1. Procédé de production d'alcools aliphatiques,
comprenant :
l'introduction d'un premier courant d'hydrocarbure contenant des oléfines linéaires et des paraffines dans une unité d'isomérisation, l'unité d'isomérisation étant conçue pour isomériser au moins une partie des oléfines linéaires contenues dans le premier courant d'hydrocarbure en oléfines ramifiées,
l'isomérisation d'au moins une partie des oléfines linéaires contenues dans le premier courant d'hydrocarbure en oléfines ramifiées dans l'unité d'isomérisation, au moins une partie des composants n'ayant pas réagi du premier courant d'hydrocarbure et au moins une partie des oléfines ramifiées produites formant un second courant d'hydrocarbure ;
l'introduction d'au moins une partie du second courant d'hydrocarbure dans une unité d'hydroformylation, l'unité d'hydroformylation étant conçue pour hydroformyler au moins une partie des oléfines contenues dans le second courant d'hydrocarbure afin de produire des alcools aliphatiques et au moins une partie des alcools aliphatiques produits comprenant un groupe alkyle ramifié, au moins une partie des composants n'ayant pas réagi du second courant d'hydrocarbure et au moins une partie des alcools aliphatiques produits formant un courant de réaction d'hydroformylation ;
la séparation d'au moins une partie du courant de réaction d'hydroformylation pour produire un courant de produit de type alcool aliphatique et un courant de paraffines et d'oléfines n'ayant pas réagi ; et
l'introduction d'au moins une partie du courant de paraffines et d'oléfines n'ayant pas réagi dans une unité de déshydrogénation, l'unité de déshydrogénation étant conçue pour déshydrogéner au moins une partie des paraffines contenues dans le courant de paraffines et d'oléfines n'ayant pas réagi afin de produire des oléfines, au moins une partie des oléfines produites sortant de l'unité de déshydrogénation pour former un courant d'hydrocarbure oléfinique ; et
l'introduction d'au moins une partie du courant d'hydrocarbure oléfinique dans l'unité d'isomérisation.

2. Procédé selon la revendication 1, dans lequel le premier courant d'hydrocarbure est produit dans un procédé d'oligomérisation d'oléfines.

3. Procédé selon la revendication 1, dans lequel le premier courant d'hydrocarbure est produit dans un procédé de Fischer-Tropsch.

4. Procédé selon l'une quelconque des revendications 1 à 3, dans lequel le premier courant d'hydrocarbure contient des oléfines et des paraffines contenant un nombre de carbone allant de 10 à 17.

5. Procédé selon l'une quelconque des revendications 1 à 4, dans lequel l'unité d'isomérisation fonctionne à une température réactionnelle située entre 200°C et 500 °C.

6. Procédé selon l'une quelconque des revendications 1 à 5, dans lequel l'unité d'isomérisation fonctionne à une pression partielle d'hydrocarbure située dans la plage allant de 10 kPa (0,1 atmosphère) à 2 026 kPa (20 atmosphères).

7. Procédé selon l'une quelconque des revendications 1 à 6, dans lequel l'unité d'hydroformylation fonctionne à une température réactionnelle allant de 100°C à 300°C.

8. Procédé selon l'une quelconque des revendications 1 à 7, comprenant en outre l'ajustement du rapport oléfines sur paraffines introduites dans l'unité d'isomérisation par l'ajout d'au moins une partie d'un courant d'hydrocarbure paraffinique dans l'unité d'isomérisation.

9. Procédé selon l'une quelconque des revendications 1 à 8, comprenant en outre:
l'ajustement du rapport oléfines sur paraffines introduites dans l'unité d'isomérisation par la combinaison d'un courant d'hydrocarbure paraffinique avec au moins une partie du premier courant d'hydrocarbure en amont de l'unité d'isomérisation pour former un courant combiné; et
l'introduction du courant combiné dans l'unité d'isomérisation.

10. Procédé selon l'une quelconque des revendications 1 à 9, comprenant en outre:
l'ajustement du rapport oléfines sur paraffines introduites dans l'unité d'isomérisation par la combinaison d'au moins une partie d'un courant d'hydrocarbure paraffinique avec au moins une partie du premier courant d'hydrocarbure en amont de l'unité d'isomérisation pour former un courant combiné ;
l'introduction du courant combiné dans l'unité d'isomérisation ;
l'ajustement du rapport oléfines sur paraffines introduites dans l'unité d'hydroformylation par la combinaison d'au moins une partie d'un troisième courant d'hydrocarbure avec au moins une partie du second courant d'hydrocarbure en amont de l'unité d'hydroformylation pour former un courant combiné ; et
l'introduction du courant combiné dans l'unité d'hydroformylation.

11. Procédé selon l'une quelconque des revendications 1 à 10, comprenant en outre l'ajustement du rapport oléfines sur paraffines introduites dans l'unité d'hydroformylation par l'ajout d'au moins une partie du troisième courant d'hydrocarbure dans l'unité d'hydroformylation.

12. Procédé selon l'une quelconque des revendications 1 à 10, comprenant en outre l'ajustement du rapport oléfines sur paraffines introduites dans l'unité d'hydroformylation par l'ajout d'au moins une partie d'un troisième courant d'hydrocarbure dans l'unité d'hydroformylation, le troisième courant d'hydrocarbure contenant plus de 80 pour cent d'oléfines en poids.

13. Procédé selon l'une quelconque des revendications 1 à 10, comprenant en outre:
l'ajustement du rapport oléfines sur paraffines introduites dans l'unité d'hydroformylation par la combinaison d'au moins une partie d'un troisième courant d'hydrocarbure avec au moins une partie du second courant d'hydrocarbure en amont de l'unité d'hydroformylation pour former un courant combiné ; et
l'introduction du courant combiné dans l'unité d'hydroformylation.

14. Procédé selon l'une quelconque des revendications 1 à 10, comprenant en outre:
l'ajustement du rapport oléfines sur paraffines introduites dans l'unité d'hydroformylation par la combinaison d'au moins une partie d'un troisième courant d'hydrocarbure avec au moins une partie du second courant d'hydrocarbure en amont de l'unité d'hydroformylation pour former un courant combiné, le troisième courant d'hydrocarbure contenant plus de 80 pour cent d'oléfines en poids ; et
l'introduction du courant combiné dans l'unité d'hydroformylation.

15. Procédé selon l'une quelconque des revendications 1 à 10, comprenant en outre:
l'ajustement du rapport oléfines sur paraffines introduites dans l'unité d'hydroformylation par la combinaison d'au moins une partie d'un troisième courant d'hydrocarbure avec au moins une partie du second courant d'hydrocarbure en amont de l'unité d'hydroformylation pour former un courant combiné, le troisième courant d'hydrocarbure contenant des oléfines linéaires ; et
l'introduction du courant combiné dans l'unité d'hydroformylation.

16. Procédé selon l'une quelconque des revendications 1 à 10, comprenant en outre:
l'ajustement du rapport oléfines sur paraffines introduites dans l'unité d'hydroformylation par la combinaison d'au moins une partie d'un troisième courant d'hydrocarbure avec au moins une partie du second courant d'hydrocarbure en amont de l'unité d'hydroformylation pour former un courant combiné, au moins une partie du second courant d'hydrocarbure contenant des oléfines ramifiées; et
l'introduction du courant combiné dans l'unité d'hydroformylation.

17. Procédé selon l'une quelconque des revendications 1 à 10, comprenant en outre:
l'ajustement du rapport oléfines sur paraffines introduites dans l'unité d'hydroformylation par la combinaison d'au moins une partie d'un troisième courant d'hydrocarbure avec au moins une partie du second courant d'hydrocarbure en amont de l'unité d'hydroformylation pour former un courant combiné, au moins une partie du troisième courant d'hydrocarbure contenant des oléfines linéaires et au moins une partie du second courant d'hydrocarbure contenant des oléfines ramifiées; et
l'introduction du courant combiné dans l'unité d'hydroformylation.

18. Procédé selon l'une quelconque des revendications 1 à 17, dans lequel l'unité de déshydrogénation fonctionne à une température située entre 300°C et 700 °C.

19. Procédé selon l'une quelconque des revendications 1 à 18, dans lequel l'unité de déshydrogénation fonctionne à une pression située entre 1 kPa (0,01 atmosphère) et 2 534 kPa (25 atmosphères).

20. Procédé selon l'une quelconque des revendications 1 à 19, comprenant en outre l'introduction d'au moins une partie du courant de produit de type alcool aliphatique dans une unité de sulfatation, l'unité de sulfatation étant conçue pour sulfater au moins une partie des alcools aliphatiques contenus dans le courant de produit de type alcool aliphatique afin de produire des sulfates aliphatiques et au moins une partie des sulfates aliphatiques produits contenant des sulfates aliphatiques ramifiés.

21. Procédé selon l'une quelconque des revendications 1 à 19, comprenant en outre l'introduction d'au moins une partie du courant de produit de type alcool aliphatique dans une unité d'oxyalkylation, l'unité d'oxyalkylation étant conçue pour oxyalkyler au moins une partie des alcools aliphatiques contenus dans le courant de produit de type alcool aliphatique afin de produire des alcools oxyalkylés, au moins une partie des alcools oxyalkylés produits contenant des alcools oxyalkylés ramifiés.
